# EUROPEAN PATENT SPECIFICATION

(11) **EP 1 726 327 B1**
(45) Date of publication and mention of the grant of the patent: **03.09.2008**
(21) Application number: 05011149.1
(22) Date of filing: 23.05.2005
(51) Int. Cl.: A61M 35/00, A45D 34/04, B65D 83/00, B05C 17/00

(54) **Sealed container with a slender inner tube to induce liquid content for an applicator**
Geschlossener Behälter mit einem länglichen Innenröhrchen zum Herausführen des flüssigen Inhalts auf einen Auftragsabschnitt
Récipient scellé muni d'un tube intérieur mince pour conduire le contenu liquide sur un applicateur

(43) Date of publication of application: 29.11.2006
(73) Proprietor: Tsaur, Garry, Rowland Heights, CA 91748 (US)
(72) Inventor: Tsaur, Garry, Rowland Heights, CA 91748 (US)
(74) Representative: Viering, Jentschura & Partner

(56) References cited:
- EP-A- 1 422 159
- WO-A-03/030681
- DE-U1-7202004 006 28

## Description

### BACKGROUND OF THE INVENTION

### 1. Field of the invention

The present invention relates to an elongated and sealed container and an applicator, more particularly, to an elongated and sealed container with a slender tube to induce the liquid content for supplying the applicator overcoming the surface tension of the liquid by rubbing against the object with the applicator.

### 2. Description of the Prior Art

Conventional types of containers for storing and transporting liquid content have several shortcomings. Most of them have to rely on externally applied force or using extra tools to extract their liquid content. Since the liquid is easy to evaporate, special care should be taken in storing liquid such as solvent or alcohol. To meet such a requirement, there are many kinds of container available on the market for storing fluids. For example a huge tank suitable for storing water. As for a small bottle for preserving a perfume, or even more tiny one for filling a special liquid. The containers are made of appropriate materials to fit for individual contents to be stored under respective circumferences.

A container according to the preamble of claim 1 is illustrated in DE 20 2004 006 287 U1.

### SUMMARY OF THE INVENTION

It is a first object of the present invention to provide a sealed container associated with an inner tube whose liquid content is kept not to flow out automatically, but instead, it must be extracted out of the container by an inner tube.

It is a second object of the present invention to provide a sealed container associated with an inner tube wherein the tube tip is affixed a tooth pick as an applicator which can be emerged out of the open port of the container so as to clean the user's mouth and teeth with the liquid contents of the inner tube such as a gargle or menthol water.

It is a third object of the present invention to provide a sealed container associated with an inner tube wherein the container is made of a soft tubular body filled with an ophthalmic solution which can be forced to flow out along its inner tube by squeezing the container.

To achieve these and other objects mentioned above, the present invention comprises the features recited in the appended claims. It comprises an elongated and sealed container which is provided with at least one prescribed frangible opening means near one end of the container filled with a liquid in a sealed state. An inner tube is coaxially interposed in the container with its one end conjoined with corresponding end of the container, while the other end is flush with the opening means or emerged out of it. An elongated tubular stump is extended from the end of the container where the opening means is provided. The container and the elongated tubular stump may be integrally fabricated in one piece, or jointed together after being separately fabricated. As soon as the container is opened, the content inside can not flow out of the container automatically by the effect of capillary repulsion and the outer atmospheric pressure. The flow out of the content along the inner tube must wait until the enclosure provided at the opening port of the container or the front end of the tubular stump contacts or rubs the object such that the surface tension of the liquid at the inner tube tip is released.

These features and advantages of the present invention will be fully understood and appreciated from the following detailed description of the accompanying Drawings.

### BRIEF DESCRIPTION OF THE DRAWINGS

Fig. 1 is a schematic view of a known container of the prior art.
Fig. 2 is a schematic view of the first embodiment according to the present invention.
Fig. 3 is a schematic view of a container which is not part of the present invention.
Fig. 4 is a schematic view of the second embodiment according to the present invention.
Fig. 5 is a schematic view of the third embodiment according to the present invention.
Fig. 6 is a schematic view of a known prior art container.
Fig. 7 is a schematic view of a known prior art container.
Fig. 8 is a schematic view of a known prior art container.

### DETAILED DESCRIPTION OF THE PREFERRED EMBODIMENT

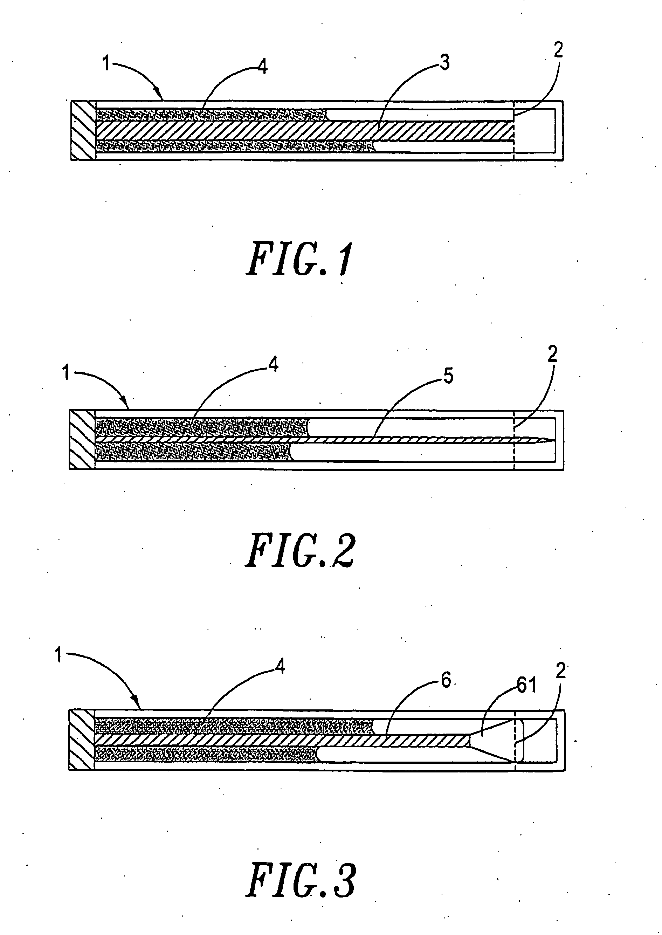

Referring to Fig. 1, a known container of the prior art comprises: an elongated and sealed container 1, a long slender inner tube 3 and a liquid 4.

The container 1 is an elongated tubular container sealed at both ends, and a prescribed frangible opening means 2 is provided near one end of the container 1. The user may open the container 1 along the scribing.

The elongated slender inner tube 3 has a diameter smaller than that of the container 1 so that it can be inserted into the container 1, and fixedly conjoin its one end with that of the container 1, and the other end of the tube 3 is aligned flush with the scribing of the opening means 3, the inner tube 3 can be either solid or hollow.

The liquid 4 filled in the container 1 in sealed state can be a solvent like perfume, alcohol, glue.

When one end of the contained is opened by breaking along the scribing of the frangible opening means 2, the length of the inner tube 3 becomes approximately equal to that of the container 1. At this moment, the liquid 4 contained in the container 1 is not yet permitted to flow out but is restrained by the capillary repulsion and the outer atmospheric pressure until the open port of the container 1 is rubbed against any object which it contacts so as to overcome the surface tension of the liquid 4 at the open port of the container 1 and extract the fluid 4 out of the container 1 along the inner tube 3 for service.

In the first embodiment of the present invention shown in Fig.2, the front tip of a slender inner tube 5 is formed into a sharp conical figure so that the inner tube 5 may serve as a tooth pick, and is emerged out of the opening means 2. The container 1 is filled with a liquid 4 such as gargle, menthol water etc. When the container 1 is opened by breaking the opening means 2 along the scribing so as to reveal the conical tip of the inner tube 5, it serves as a tooth pick. At this very moment, having been broken the surface tension, the liquid 4 is extracted out of the container 1 along the inner tube 5 into the user's mouth by gravity thereby cleaning the user's mouth.

In the container shown in Fig 3, an elongated slender inner tube 6 is laid along the container 1 slightly retreated from the opening means 2, and the end nearby the opening means 2 is wrapped with an absorber 61 which is protruded from the position of the opening means 2. The container 1 is filled with a volatile liquid 4 such as alcohol, perfume etc. When the container 1 is opened by breaking the opening means 2 along the scribing so as to open the container 1 at its one end, the absorber 61 emerges out of the opening port of the container 1. When the absorber 61 emerges out of the opening port of the container 1 is rubbed with any object the surface tension of the liquid 4 is broken and it flows out of the container 1 along the inner tube 6 by gravity. Then the flowed out liquid 4 is absorbed by the absorber 61 which can now serve as an applicator.

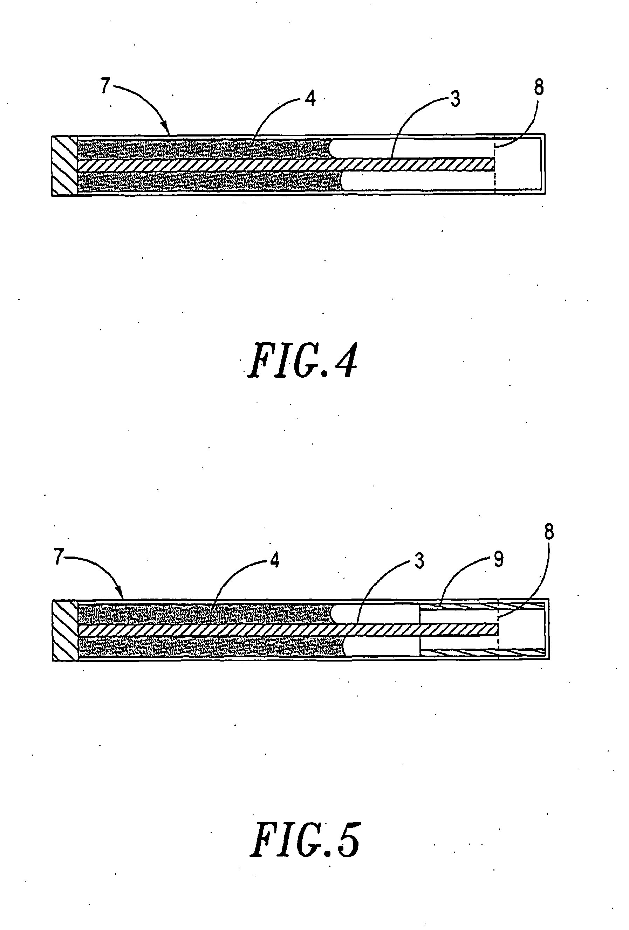

In the second embodiment of the present invention shown in Fig. 4, a container 7 is formed into an elongated soft tube, and has an elongated slender inner tube 3 as that similar to the former embodiment. One end of the inner tube 3 is fixedly conjoined with the flexible tubular container 7, while the other end thereof is aligned flush with the opening means 8. The soft tubular container 7 is filled with a liquid 4 such as ophthalmic solution or artificial tear. When the container 7 is opened at one end by breaking the opening means 8 along its scribing, the liquid 4 is still confined in the container 7 by the capillary repulsion and the outer atmospheric pressure unless the container 7 is squeezed by an extraneous force so as to overcome the surface tension of the liquid 4 and let it flow out of the container 7 along the inner tube 3. If the applied squeezing force is released, the liquid 4 stops flowing out and is again restrained in the container 7 by the capillary repulsion and the atmospheric pressure. With this structure, the user must continuously squeeze the container 7 to extract the liquid 4 drop by drop for ophthalmic use.

In the third embodiment of the present invention shown in Fig. 5, a hollow plug 9 is plugged into the container 7 at the position overlaying the opening means 8 for reducing the inner space of the soft container 7. The hollow plug 9 will be opened by breaking the scribing of the opening means 8 such that an open port with a smaller cross sectional area than that of the container 7 can be obtained. With this meaner, the flow of liquid 4 is effectively controlled.

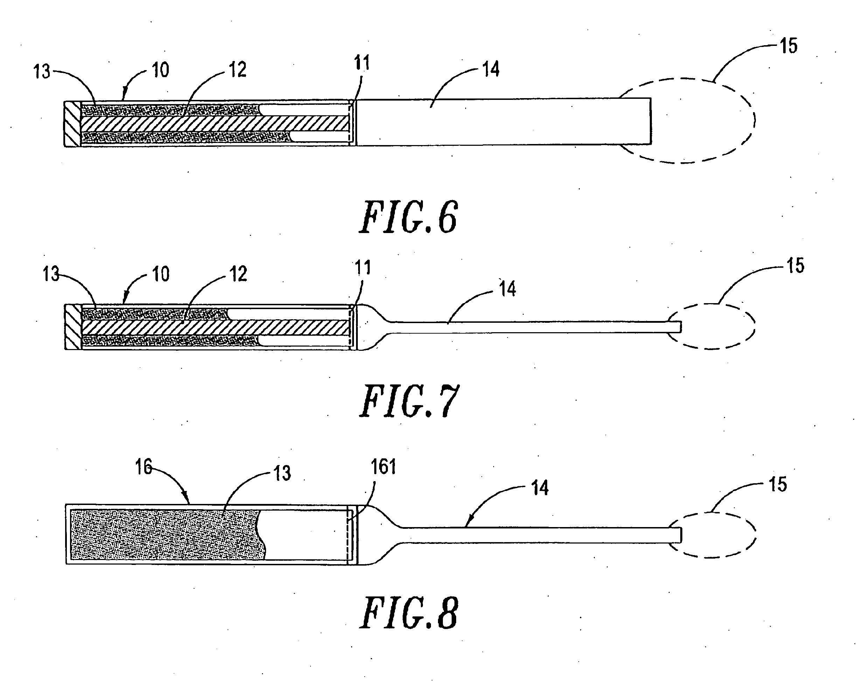

The known prior art container shown in Fig. 6, comprises: an elongated and sealed container 10, a long slender inner tube 12, a liquid 13, and a tubular stump 14.

The container 10 is an elongated tubular container and sealed at both ends, and a prescribed frangible opening means 11 is provided near one end of the container 10. The user may cut to open the container 10 along the scribing.

The elongated slender inner tube 12 has a diameter smaller than that of the container 10 so that it can be inserted into the container 10, and fixedly conjoin its one end with that of the container 10, and the other end of the tube 12 is aligned flush with the scribing of the opening means 11, the inner tube can be either solid or hollow.

The liquid 13 is filled in the container 10 in sealed state.

The tubular stump 14 is a solid or hollow elongated tube with its one end integrally fabricated in one piece with one end of the container 10 where exists the opening means 11, while the other end thereof is formed into an enclosure 15 such as a cotton swab, a spoon or a brush serving as an applicator. Alternatively the tubular stump 14 and the container 10 can be jointed end to end after the two are separately fabricated.

The user's hand or fingers does not have to touch the enclosure 15 when he/she is opening the container 10 so that the enclosure 15 is never being contaminated. The front tip of the inner tube 12 aligns flush with the open port of the container 10 when the scribing of the opening means 11 is broken. At this moment, being retrained by the capillary repulsion and the outer atmospheric pressure, the liquid 13 is still unable to flow out until the enclosure 15 of the tubular stamp 14 is rubbed with the open port of the container 10 to break the surface tension of the liquid 13 thereat thereby enabling the liquid 13 to flow out of the container 10 along the inner tube 12 with the aid of gravity to be absorbed by the enclosure 15 for serving as an applicator.

In the known prior art container shown in Fig. 7, here, the container 10 and the tubular stump 14 is configurated into different shape from that described in the previous embodiments. As shown in Fig. 7, the diameter of the sealed container 10 is made larger than that of the tubular stump 14 or vice versa.

In the known prior art shown in Fig. 8, here, a sealed container 16 is formed of a long sealed tubular body with a large diameter to retain the liquid 13 in sealed state. One or more prescribed opening means 161 is provided near its end portion. An elongated solid or hollow tubular stump 14 is formed integrally in one piece with the end portion of the container 16 and has an enclosure 15 affixed to the front tip thereof. The enclosure 15 may be a cotton swab a spoon or a brush serving as an applicator. The user's hand or fingers does not have to touch the enclosure 15 when he/she is opening the container 16 so that the enclosure 15 is never being contaminated. As soon as the container 16 is opened at one end by breaking the scribing of the opening means 161, the liquid 13 flows along the tubular stump 14 to drain the enclosure 15 for serving as an applicator.

In brief, it emerges from the description of the above embodiments that the invention has several noteworthy advantages compared with any like products, in particular:
1.That the ingeniously designed inner tube induces the inner liquid to flow out slowly for application instead of pouring out from the container.
2.That a tooth pick like applicator formed at the front tip of the inner tube can be served associated with the content of a gargle, or menthol solution for cleaning the user's mouth.
3.That the absorber provided at the front tip of the inner tube emerged out of the open port of the container can be used to absorb the inner liquid by rubbing against an object so as to serve as an applicator.
4.That the container may be formed of the soft tube to accommodate ophthalmic solution for medical use by squeezing the container to apply the content to the patient's eyes.
5.That the applicator extended out of the end portion of the container is free from being contacted with the user's hand or finger's thereby eliminating the fear of spoiling the applicator.
6.That the content never flows out automatically when the container is opened, but it slowly flows out along the inner tube after the applicator is contacted with the open port of the container.
7.That the content of the container can be directly supplied to the applicator if the container is formed of a coarse tube.
8.That many kinds of elements can be selected to serve as an applicator such as, a cotton swab, a spoon or a brush.
9.That the sealed container may be configurated into various shapes.

A variety of modifications and variations of the present invention are possible in light of the above teachings. It is therefore to be understood that within the scope of the appended claims, the present invention may be practiced otherwise than as specially described hereinabove.

## Claims

1. A sealed container comprising:
an elongated tubular container (1, 7) sealed at both ends, and having at least one prescribed frangible opening means (2, 8) provided near its one end thereof;
an elongated slender inner tube (3, 5) inserted into said container (1,7) and fixedly conjoining its one end with that of said container (1, 7); and
a liquid (4) filled in said container (1, 7) in sealed state;
**characterized in that**
after the surface tension of said liquid has been overcome, said liquid (4) flows out along said inner tube (3, 5) with the aid of gravity when one end of said container (1, 7) is opened by opening said opening means (2, 8); and
a) wherein a front tip of the inner tube (5) is formed into a sharp conical figure and is emerged out of said opening means so that the inner tube (5) is able to serve as a toothpick, or
b) wherein said container (7) is formed into an elongated soft tube able to be squeezed.

2. The sealed container as in claim 1, wherein said opening means (2, 8) is a scribing to open said container (1, 7) by cutting along said scribing.

3. The sealed container as in claim 1, alternative a), wherein when said container (1) is opened by said opening means (2), said front tip of said inner tube (5) is emerged out of the open port of said container (1) so as to overcome the surface tension of the liquid (4) at the open port of said container (1) thereby introducing the liquid (4) to flow out along said inner tube (5) with the aid of gravity.

4. The sealed container as in claim 3, wherein the liquid (4) in said container (1) is a gargle, a menthol solution, or a sterilizing water.

5. The sealed container as in claim 1, alternative b), wherein when said soft container (7) is opened by said opening means (8), one end of said inner tube (3) is aligned flush with the open port of said container (1) so that when said soft container (7) is squeezed, the surface tension of said liquid (4) at the open port is broken by said inner tube (3) thereby said liquid (4) is able to flow out along said inner tube (3) with the aid of gravity.

6. The sealed container as in claim 5, wherein said liquid (4) stored in said soft container (7) is an ophthalmic drug, an artificial tear, or liquidal glue.

7. The sealed container as in claim 1, alternative b), wherein a hollow plug (9) is plugged into said soft container (7) at the position overlaying said opening means (8) for reducing the inner volume of said soft container (7) such that the cross sections at two ends of said soft container (7) become different from each other thereby the flow of said liquid (4) is effectively controllable.

8. The sealed container as in claim 7, wherein said plug (9) has a similar prescribed opening means (8) overlaying that of said container (7) to be simultaneously opened.

## Patentansprüche

1. Geschlossener Behälter, aufweisend:
einen verlängerten rohrförmigen Behälter (1, 7), der an beiden Enden geschlossen ist und wenigstens ein vorgeschriebenes zerreißbares Öffnungsmittel (2, 8) aufweist, das nahe seinem einen Ende davon vorgesehen ist;
ein verlängertes schmales Innenröhrchen (3, 5), das in den Behälter (1, 7) eingesetzt ist und dessen eines Ende mit dem des Behälters (1, 7) fest verbunden ist; und
eine Flüssigkeit (4), die in dem Behälter (1, 7) im geschlossenen Zustand eingefüllt ist;
**dadurch gekennzeichnet, dass**
nachdem die Oberflächenspannung der Flüssigkeit überwunden wurde, die Flüssigkeit (4) mit Hilfe von Schwerkraft entlang dem Innenröhrchen (3, 5) heraus fließt, wenn das eine Ende des Behälters (1, 7) durch Öffnen des Öffnungsmittels (2, 8) geöffnet wird; und
a) wobei eine vordere Spitze des Innenröhrchens (5) zu einer spitzen konischen Gestalt geformt ist und aus dem Öffnungsmittel heraustritt, so dass das Innenröhrchen (5) geeignet ist, als ein Zahnstocher zu dienen, oder
b) wobei der Behälter (7) zu einem verlängerten weichen Rohr geformt ist, das geeignet ist, gequetscht zu werden.

2. Geschlossener Behälter nach Anspruch 1, wobei das Öffnungsmittel (2, 8) ein Anriss ist, um den Behälter (1, 7) durch Schneiden entlang dem Anriss zu öffnen.

3. Geschlossener Behälter nach Anspruch 1, Alternative a), wobei, wenn der Behälter (1) durch das Öffnungsmittel (2) geöffnet wird, die vordere Spitze des Innenröhrchens (5) aus der offenen Mündung des Behälters (1) derart heraustritt, dass die Oberflächenspannung der Flüssigkeit (4) an der offenen Mündung des Behälters (1) überwunden wird, wodurch die Flüssigkeit (4) mit Hilfe von Schwerkraft entlang dem Innenröhrchen (5) heraus fließen kann.

4. Geschlossener Behälter nach Anspruch 3, wobei die Flüssigkeit (4) in dem Behälter (1) ein Gurgelmittel, eine Menthollösung oder ein Sterilisationswasser ist.

5. Geschlossener Behälter nach Anspruch 1, Alternative b), wobei, wenn der weiche Behälter (7) durch das Öffnungsmittel (8) geöffnet wird, das eine Ende des Innenröhrchens (3) fluchtend zu der offenen Mündung des Behälters (1) ausgerichtet ist, so dass, wenn der weiche Behälter (7) gequetscht wird, die Oberflächenspannung der Flüssigkeit (4) an der offenen Mündung durch das Innenröhrchen (3) unterbrochen wird, wodurch die Flüssigkeit (4) in der Lage ist, mit Hilfe von Schwerkraft entlang dem Innenröhrchen (3) herauszufließen.

6. Geschlossener Behälter nach Anspruch 5, wobei die in dem weichen Behälter (7) gespeicherte Flüssigkeit (4) ein Augenmedikament, eine künstliche Träne oder ein Flüssigklebstoff ist.

7. Geschlossener Behälter nach Anspruch 1, Alternative b), wobei ein hohler Stopfen (9) in den weichen Behälter (7) in der dem Öffnungsmittel (8) überlagerten Position zum Reduzieren des Innenvolumens des weichen Behälters (7) derart eingesteckt ist, dass die Querschnitte an beiden Enden des weichen Behälters (7) voneinander abweichen, wodurch der Fluss der Flüssigkeit (4) wirksam steuerbar ist.

8. Geschlossener Behälter nach Anspruch 7, wobei der Stopfen (9) ein gleichartiges vorgeschriebenes Öffnungsmittel (8) aufweist, das dem des Behälters (7) überlagert ist, um gleichzeitig geöffnet zu werden.

## Revendications

1. Récipient étanche comprenant :
un récipient (1, 7) tubulaire allongé fermé hermétiquement aux deux extrémités, et comportant au moins un moyen d'ouverture fragile (2, 8) prescrit prévu à proximité d'une extrémité de celui-ci ;
un tube intérieur plus fin (3, 5) allongé inséré dans ledit récipient (1, 7) et dont une extrémité est liée fermement à celle dudit récipient (1, 7) ; et
un liquide (4) introduit dans ledit récipient (1, 7) dans un état étanche ;
**caractérisé en ce que**
une fois que la tension superficielle dudit liquide a été surmontée, ledit liquide (4) s'écoule le long dudit tube interne (3, 5) par la gravité lorsqu'une extrémité dudit récipient (1, 7) est ouverte en ouvrant ledit moyen d'ouverture (2, 8) ; et
a) dans lequel un bout avant du tube interne (5) est réalisé en une forme conique pointue et émerge dudit moyen d'ouverture de sorte que le tube interne (5) est capable de servir en tant que cure-dent, ou
b) dans lequel ledit récipient (7) est formé en un tube souple allongé pouvant être pressé.

2. Récipient étanche selon la revendication 1, dans lequel ledit moyen d'ouverture (2, 8) est une découpe pour ouvrir ledit récipient (1, 7) par découpage le long de ladite découpe.

3. Récipient étanche selon la revendication 1, variante a), dans lequel, lorsque ledit récipient (1) est ouvert par ledit moyen d'ouverture (2), ledit bout avant dudit tube interne (5) émerge de l'orifice ouvert dudit récipient (1) de manière à surmonter la tension superficielle du liquide (4) au niveau de l'orifice ouvert dudit récipient (1), amenant de ce fait le liquide (4) à s'écouler le long dudit tube interne (5) par la gravité.

4. Récipient étanche selon la revendication 3, dans lequel le liquide (4) dans ledit récipient (1) est un gargarisme, une solution mentholée, ou une eau de stérilisation.

5. Récipient étanche selon la revendication 1, variante b), dans lequel, lorsque ledit récipient souple (7) est ouvert par ledit moyen d'ouverture (8), une extrémité dudit tube interne (3) est alignée au niveau de l'orifice ouvert dudit récipient (1) de sorte que, lorsque ledit récipient souple (7) est pressé, la tension superficielle dudit liquide (4) au niveau de l'orifice ouvert est rompue par ledit tube interne (3), ledit liquide (4) étant de ce fait capable de s'écouler le long dudit tube interne (3) par la gravité.

6. Récipient étanche selon la revendication 5, dans lequel ledit liquide (4) stocké dans ledit récipient souple (7) est un médicament ophtalmique, une larme artificielle, ou une colle liquide.

7. Récipient étanche selon la revendication 1, variante b), dans lequel un bouchon creux (9) est introduit dans ledit récipient souple (7) à la position recouvrant ledit moyen d'ouverture (8) pour réduire le volume interne dudit récipient souple (7) de sorte que les sections aux deux extrémités dudit récipient souple (7) deviennent différentes l'une de l'autre, l'écoulement dudit liquide (4) pouvant de ce fait être contrôlé efficacement.

8. Récipient étanche selon la revendication 7, dans lequel ledit bouchon (9) comporte un moyen d'ouverture (8) prescrit similaire recouvrant celui dudit récipient (7) pour qu'ils soient ouverts simultanément.
